(19) Europäisches Patentamt
European Patent Office
Office européen des brevets

(11) **EP 2 260 880 A1**

(12) **EUROPEAN PATENT APPLICATION**

(43) Date of publication:
**15.12.2010 Bulletin 2010/50**

(21) Application number: **10165564.5**

(22) Date of filing: **10.06.2010**

(51) Int Cl.:
*A61L 27/34* (2006.01)    *A61L 29/08* (2006.01)
*A61L 31/10* (2006.01)    *A61L 33/00* (2006.01)
*A61L 33/08* (2006.01)

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **12.06.2009 US 186524 P**

(71) Applicant: **Greatbatch Ltd.
Clarence, New York 14031 (US)**

(72) Inventor: **Ye, Qingshan
Plymouth, MN 55441 (US)**

(74) Representative: **Koepe & Partner
Patentanwälte
Robert-Koch-Strasse 1
80538 München (DE)**

(54) **Biomimetic coating method**

(57)    A modified method of preparing and applying a biomimetic coating to a medical device substrate surface is described. The modified biomimetic coating method utilizes a solvent mixture of water and an organic water miscible solvent that results in a more efficient coating process, reducing the time required to apply a sufficiently adherent biomimetic coating.

| | |
|---|---|
| STEP 1 | Clean substrate surface. |
| STEP 2 | Prepare biomimetic coating solution by dissolving the biomimetic surfactant in a water miscible solvent/water mixture. |
| STEP 3 | Apply biomimetic solution to substrate surface and dry. |
| STEP 4 | Clean coated substrate surface. |
| STEP 5 | Heat treat biomimetic coated surface. |

FIG. 2

**Description**

CROSS-REFERENCE TO RELATED APPLICATION

The present application claims priority from U.S. Provisional Application Serial No. 61/186,524, filed June 12, 2009.

BACKGROUND OF THE INVENTION

Field of the Invention

The present invention is related generally to biocompatible coatings. More specifically, the present invention is related to a method of applying a biomimetic coating to a medical device surface.

BACKGROUND ART

Implantable medical devices including, pacemakers, defibrillators, neurostimulators, venous introducers, and catheters are devices that are well known to help improve health and sustain life. However despite the significant benefits that implantable medical devices provide, their use could lead to thrombosis, a serious medical problem that could result in death. Thrombosis is the formation of a blood clot within a blood vessel that obstructs blood flow leading to possible stroke, heart attack, organ failure and death.

Medical device related thrombosis initially occurs as a result of an interaction between blood and the surface of the medical device when they are in contact with each other. Once blood is in contact with the medical device, blood platelets and other blood constituents begin to coagulate and clot on the device surface. Blood clotting is known to occur on both metallic and polymeric materials which are used to manufacture medical devices.

After the formation of the blood clots have occurred on the device surface, the clots could break off from the surface, travel through the blood stream, become lodged in a blood vessel and obstruct blood flow. Thrombosis is an especially major problem for permanently implanted devices that are in continuous blood contact.

A great deal of work has been done to develop coatings which reduce cell adhesion and activation. These coatings, referred to as biomimetic coatings, can inhibit the formation of blood clots and therefore reduce the possibility of thrombosis from occurring.

One such family of biomimetic coatings are surfactants described by Marchant et al. in U.S. patents 6,759,388 and 7,276,474 and U.S. patent application publications 20080247988 and 20080262614, which are herein incorporated by reference.

These coatings provide good blood clot inhibition. However, current processing and coating preparation methods require a considerable amount of time that is not ideal for cost effective manufacturing.

An objective of the present invention, therefore, is to provide an improved efficient means of applying the biomimetic surfactant to a medical device surface.

SUMMARY OF THE INVENTION

The present invention relates to modification of the method of preparing a biomimetic material for application onto the surface of a medical device. Biomimetic surfactants, which will be discussed in more detail, are designed to reduce protein, platelet and leukocyte adhesion, and as a result, reduce the likelihood of thrombosis.

The biomimetic surfactant coatings developed by Marchant et al. are known to reduce platelet adhesion and activation on the coating surface. It is currently understood that these biomimetic surfactant materials must be combined with water to create an entirely aqueous biomimetic solution which is then applied to a substrate surface. As Marchant states in column 6 of the '388 patent the "surfactant polymers of the present invention, preferably, are soluble in water and are easily applied to the surface of the biomaterial. Application is achieved by immersing the biomaterial in a solution, preferably an aqueous solution, comprising the surfactant polymer. The surfactant spontaneously attaches to the hydrophobic surface of the polymeric biomaterial to provide a monolayer which alters the surface properties of the hydrophic surface." Marchant further states in the '388 patent that "a monolayer of the surfactant polymer of example 2 was also attached to a low density polyethylene substrate by immersing the substrate in an aqueous solution comprising 1-2 mg/ml of the surfactant polymer for 24 hours. Thereafter the monolayer was air dried..."

However, a series of experiments have been performed that negates this commonly held understanding that an entirely aqueous solution of these biomimetic surfactant materials is required in order to achieve sufficient coating adhesion. Furthermore, the prior method of creating an aqueous biomimetic surfactant solution procedure requires that the surface be submerged in the solution for at least 24 hours to achieve a sufficiently adherent coating. Such a coating procedure requiring at least 24 hours of submersion in a biomimetic solution is not conducive to a viable cost effective manufacturing process. Therefore, it is desirable to modify the current aqueous biomimetic coating solution procedure to improve coating process efficiency to achieve a sufficiently adherent biomimetic coating that is more cost effective and manufacturable.

The improved method is mainly directed to a process of applying a biomimetic coating onto the surface of a polyurethane substrate, however, other common medical device substrate polymeric and metallic materials such as silicone, polytetrafluoroethylene, polyether block amides, manufactured under the "Pebax" trade name, stainless steel, titanium, and MP35N can also be readily coated using the present method.

These materials are often used in the construction of a wide range of medical devices that include catheters, intravenous introducers, pacemakers, defibrillators, neurostimulators, and their associated leads.

Therefore, it is desirable to modify the preparation method of the biomimetic coating so as to reduce the time required to produce an acceptably adherent biomimetic coating that is more cost effective and manufacturable.

In the present invention, an organic water miscible solvent is added to create a solvent mixture comprising the solvent and water combination in which the biomimetic surfactant is dissolved.

The term "biomimetic" is defined herein as mimicking bodily cell interaction at the molecular level so as not to cause an adverse affect or reaction in the body. The term "non-thrombogenic" is defined herein as prohibiting the coagulation of blood from occurring in a blood vessel. The term "water miscible" is defined herein as dissolvable in water. The term "substrate" is defined herein as a base material which can be modified through the application of a surface coating or through the incorporation of dopant materials during processing of the base material. As referred to in this present invention, a substrate surface refers to the surface of a medical device.

Once the substrate surface has been sufficiently modified, as described in U.S. patent application serial number 12/776,095, incorporated by reference herein, a biomimetic surfactant polymer such as poly(N-vinyldextran aldonamide-co-N-vinylhexanamide) or its derivatives is applied onto the modified substrate surface utilizing the present method.

The biomimetic surfactant comprises a polymeric backbone of repeating monomeric units having functional groups for coupling with side chains. The surfactant comprises two main functional groups, a hydrophobic side chain functional group and a hydrophilic side chain functional group. The hydrophobic side chain affects the bonding adhesion of the surfactant to the substrate surface. The hydrophilic side chain functional group controls the biomimetic properties of the surfactant and creates an effective non-thrombogenic surface that retards blood clotting.

As will be discussed in more detail, the organic water miscible solvent component comprised in the solvent mixture of the present method, alters the solubility of the biomimetic surfactant and allows the surfactant to more readily attach to the substrate surface. Thus the time required to produce a sufficiently adherent biomimetic coated surface is significantly reduced.

BRIEF DESRIPTION OF THE DRAWINGS

FIG. 1 is a depiction showing the chemical structure of the preferred biomimetic surfactant used in the present invention.

FIG. 2 is a flow chart showing the sequence of the method of the present invention.

DETAILED DESCRIPTION OF THE PREFERRED EMBODIMENTS

The present invention details an improved method of preparing a biomimetic coating that results in a more efficient coating application on a substrate surface. In a preferred embodiment, the biomimetic surfactant poly(N-vinyldextran aldonamide-co-N-vinylhexanamide) is applied to the surface of a medical device using the present invention to reduce the occurrence of thrombosis.

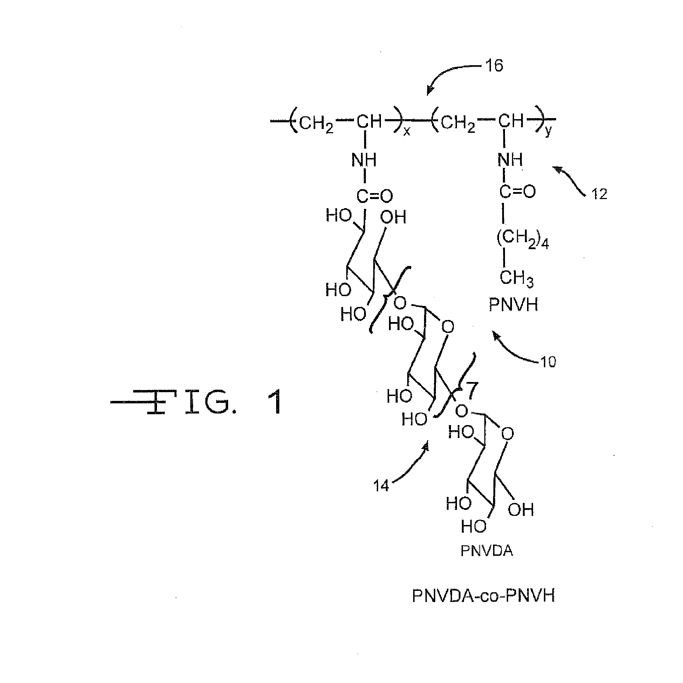

As shown in FIG. 1, the biomimetic surfactant comprises a chemical structure 10 that is composed of a combination of a hydrophobic molecular chain and a hydrophilic molecular chain. The hydrophobic molecular chain comprises a poly(N-vinyl hexanoyloxy) (PNVH) component 12 and the hydrophilic molecular chain comprises a poly(N-vinyl dextran aldonamide) (PNVDA) component 14. The molecular weight of the preferred surfactant ranges from about 1,000 to about 2,000,000 dalton.

The biomimetic surfactant is preferably applied to the substrate surface through the application of a coating. It is preferred that a polyurethane substrate surface be coated. However, other polymeric substrate materials not limited to silicone, polytetrafluoroethylene, polyether block amide, and polyetheretherketone can also be coated. The present method is not limited to coating the surfaces of polymers. Instead, the present method also enables one to apply the biomimetic surfactant to metallic surfaces not limited to stainless steel, MP35N, titanium, gold, platinum, palladium, and silver.

When applying the biomimetic coating to these alternate material surfaces, a layer of polyurethane, silicone or polyether block amide must first be applied to the polymeric or metallic surface before the biomimetic coating is applied. Therefore, it is contemplated that the polymeric and metallic surfaces of such medical devices not limited to pacemakers, defibrillators, neurostimulators, introducers, leads, catheters and stents can be modified with a biomimetic coating using the present method.

Alternate biomimetic surfactants comprising poly(N-vinyl dextran aldonamide-co-N-vinyl dodecanoamide)(PNVDA-co-PNVL), poly (N-vinyl hexyl amine-co-N-vinyl heparinamine)(PNVHA-co-PNVHep A), poly(N-vinyl hexyl amine-co-N-vinyl heparinamine-coN-vinyl maltonoamide)(PNVHA-co-PN-VHepA-co-PNVM), and poly (N-vinyl-5-peptidyl-pentylamine-co-N-vinyl-dextranaldonamine-co-N-vinyl hexyl amine (PVAm(Pep:Dex:Hex)), can also be utilized in the present process as well. Furthermore, fluorocarbon based biomimetic surfactants such as poly(*N*-vinyldextranaldonamide-*coN*-vinylperfluoroundecanamide) can be utilized with the present invention.

As illustrated in Fig. 1, the preferred biomimetic surfactant has a comb-like structure that comprises a flexible polymeric backbone 16 that is linked to a combination of a plurality of hydrophobic side chains, poly(N-vinyl hexanoamide) (PNVH) 12 and a plurality of hydrophilic side chains, poly(N-vinyl dextran aldonamide) (PNVDA), 14.

The hydrophobic side chains 12 comprise alkyl groups that are linked to the polymeric backbone 16 via an ester linkage, an amine linkage or an amide linkage. Preferably, the hydrophobic chains 12 are attached to the polymeric backbone 16 by reacting an alkanoyl ($CH_3(-CH_2-)nCO-$) or an alkanal ($CH_3(CH_2-)nCHO$) with the homopolymer of the backbone.

In the present invention, the biomimetic surfactant material is dissolved in a solvent mixture of water and an organic water miscible solvent, preferably comprising isopropyl alcohol (IPA). The solvent mixture preferably comprises an isopropyl alcohol and water solvent mixture of about 10 to 70 volume percent isopropyl alcohol, more preferably about 40 to 60 volume percent isopropyl alcohol and most preferable about 50 volume percent isopropyl alcohol.

Furthermore other organic water miscible solvents can be substituted for the isopropyl alcohol component in the mixture. Such water miscible solvents include, but are not limited to, methanol, ethanol, 1-propanol, 2-propanol, acetonitrile, *N,N*-dimethylformamide and tetrahydrofuran could also be mixed with the water to create the solvent. This organic water miscible solvent component improves the biomimetic coating efficiency on the surface of the medical device.

The content of the water miscible solvent component, such as isopropyl alcohol, alters the distribution of the biomimetic surfactant between the solution phase and the substrate surface phase. This alteration decreases the distribution of the biomimetic surfactant in the solution that drives the biomimetic surfactant material out of the solution to the surface of the substrate.

The effect of the water miscible solvent component, i.e. the isopropyl alcohol, increasing the coating efficiency can be described by the partition or distribution coefficient equation (Equation 1). The partition coefficient equation describes the equilibrium distribution of two immiscible phases, such as a gas and liquid, as they interact in a heterogeneous equilibrium as shown below.

$$K_{partition} = \frac{\left[ X_{(phase\,1)} \right]}{\left[ X_{(phase\,2)} \right]} \quad (Equation\ 1)$$

In the present invention, the principle of the equation can be utilized to illustrate the equilibrium between the biomimetic surfactant solution, and the substrate surface (Equation 2).

$$K_{partition} = \frac{[Surfactant]_{\frac{IPA}{water}}}{[Substrate\,Surface]} \quad (Equation\ 2)$$

As can be seen from Equation 2 above, the partition coefficient describes the dynamic equilibrium between the biomimetic surfactant solution of an isopropyl alcohol and water mixture and the substrate surface. Coefficient K, a unit less number, equates to the rate of diffusion of the surfactant material to the substrate surface in achieving a balanced equilibrium between the two phases.

The addition of the isopropyl alcohol or similar water miscible organic solvent of the present invention decreases the solubility of the biomimetic surfactant in the solvent mixture, as compared to its solubility in pure water. That drives the biomimetic surfactant material from the solvent to the substrate surface, creating a coating of biomimetic surfactant and establishing an equilibrium between the phases of the solution and substrate surface. The adhesion of the surfactant material from the solvent mixture to the substrate surface is increased, resulting in an increase in the efficiency of the biomimetic coating process.

In step one of the present method, the substrate surface is initially cleaned with an isopropyl alcohol and water solvent mixture. In a preferred embodiment, the solvent mixture comprises about 80 to about 95 volume percent isopropyl alcohol. After cleaning the substrate surface with the isopropyl alcohol and water solvent mixture, the surface is then rinsed under de-ionized water for about one to two minutes and dried in ambient air.

In step two of the present method, the biomimetic coating solution is prepared. In a preferred embodiment, about one weight percent of the biomimetic surfactant is dissolved in a solvent mixture comprising about 50:50, by volume, water and an organic water miscible solvent, preferably isopropyl alcohol as previously detailed. In an alternately preferred embodiment, about 0.1 to about 20 weight percent of the biomimetic surfactant material is dissolved in the solvent mixture.

In step three of the present method, the biomimetic solution is applied to the substrate surface, forming a coating of the biomimetic surfactant material on the surface. In a preferred embodiment, the surface is dipped into the solution for about one to two minutes to achieve a coating thickness of about 1 nm to about 10 nm. Alternatively, the biomimetic solution can be applied to the substrate surface using an alternate means such as spray coating, spin coating or by brushing the solution onto the surface. Multiple layers of the biomimetic surfactant material can be applied to the substrate surface.

Once the surface is coated, it is then left to dry in ambient air for about 15 to 25 minutes making sure to dry any crevices or inner surfaces with compressed air.

In step four of the present method, after the biomimetic coating has been applied and dried, the coated surface is then cleaned. The coated surface is preferably dipped or rinsed with de-ionized water. In a preferred embodiment, the surface is sequentially dipped in at least two baths of de-ionized water. The surface is dipped in a first bath of agitated de-ionized water for about 3 to 5 minutes to remove any loosely bound surfactant particles. Following the first bath, the surface is then dipped in a second bath of agitated de-ionized water bath for an additional one to two minutes to remove any residual surfactant particles that may not have been removed from the first bath. The surface may also be rinsed with de-ionized water at a preferred flow rate of about 1 mL/min to about 10 mL/min to remove any non-adherent surfactant particles. Once the surface has been rinsed, the surface is then blow dried with filtered compressed air.

In step five of the present method, the final step of the process, the coated surface is heat treated at a temperature about 30°C to about 55°C for about 30 to 60 minutes.

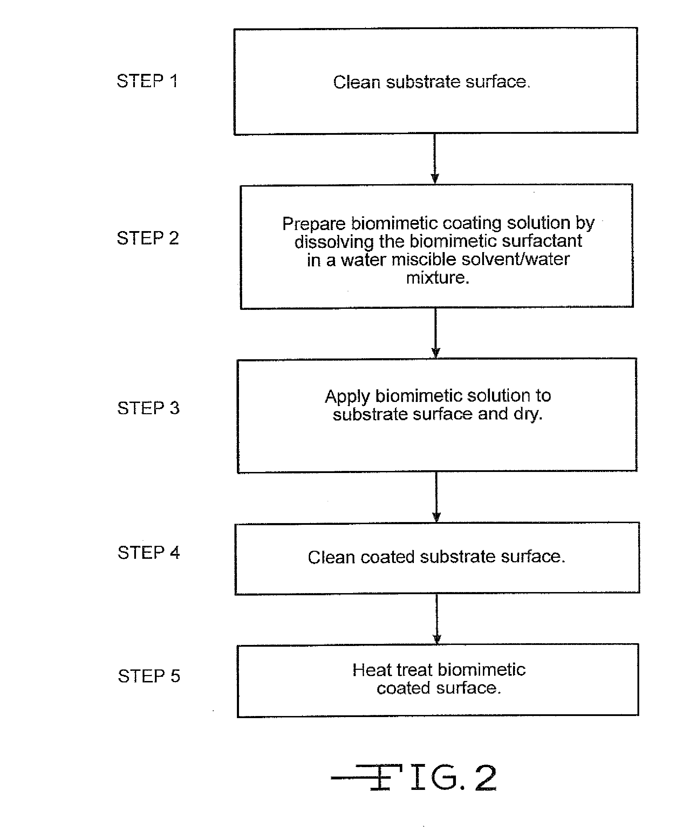

A flow chart diagram of the present procedure is shown in FIG. 2. This diagram presents an overview of the steps required in the present invention that achieves an adequately adherent biomimetic coating in about 2 hours as compared to the previous 24 hours of processing time.

Thus, the present invention teaches an improved, more efficient method of applying the biomimetic surfactant onto the medical device surface. The thusly modified processing method greatly reduces the time required for a biomimetic coating to adhere onto the polymeric substrate and prevent the formation of blood clots. This makes the modified biomimetic coating processing method a desirable candidate for use in the manufacture of implantable medical devices.

**Claims**

1. A method for providing a biomimetic coating on a substrate surface, the method comprising:

   a) providing a substrate with a surface to be coated;
   b) providing a biomimetic surfactant;
   c) mixing the biomimetic surfactant in a solvent mixture comprised of water and an organic water miscible solvent, thereby creating a biomimetic surfactant solution; and
   d) applying the biomimetic surfactant solution to the substrate surface, wherein the organic water miscible solvent drives the biomimetic surfactant out of solution onto the substrate surface creating a coating of biomimetic surfactant.

2. The method of claim 1, wherein the biomimetic material is a surfactant selected from the group consisting of poly (N-vinyldextran aldonamide-co-N-vinylhexanamide) (PNVDA-co-PNVH), poly(N-vinyl dextran aldonamide-co-N-vinyl dodecanoamide)(PNVDA-co-PNVL), poly (N-vinyl hexyl amine-co-N-vinyl heparinamine) (PNVHA-co-PNVHep A), poly(N-vinyl hexyl amine-co-N-vinyl heparinamine-co-N-vinyl maltonoamide)(PNVHA-co-PN-VHepA-co-PNVM), poly (N-vinyl-5-peptidyl-pentylamine-co-N-vinyldextran aldonamine-co-N-vinyl hexyl amine) (PVAm(Pep:Dex:Hex)), and poly (*N*-vinyldextranaldonamide-*co-N*-vinylperfluoroundecanamide).

3. The method of claim 1, wherein the substrate surface is selected from the group consisting of polyurethane, silicon, polyether block amide (PEBA), polycarbonate, polytetrafluoroethylene, polyetheretherketone, stainless steel, titanium, MP35N, gold, platinum, palladium, silver, and combinations thereof.

4. The method of claim 1, wherein the organic water miscible solvent is selected from the group consisting of isopropyl alcohol, methanol, ethanol, 1-propanol, 2-propanol, acetonitrile, $N,N'$-dimethylformamide and tetrahydrofuran.

5. The method of claim 1 wherein the solvent mixture comprises 10 to 70 volume percent organic water miscible solvent; preferably wherein the solvent mixture comprises 40 to 60 volume percent organic water miscible solvent; more preferably wherein the solvent mixture comprises 50 volume percent organic water miscible solvent.

6. The method of claim 1, wherein the concentration of the biomimetic surfactant is from 0.1 to 20 weight percent, and/or wherein the thickness of the biomimetic coating is from 1 nm to 10 nm.

7. The method of claim 1, wherein the biomimetic coating is applied to the substrate surface from the group of application methods consisting of dipping, spray coating, spin coating, and brushing.

8. The method of claim 1, wherein the substrate surface is a surface selected from the group of medical devices consisting of a pacemaker, a defibrillator, a neurostimulator, an introducer, a lead, a catheter, and a stent.

9. A method for providing a biomimetic coating on a substrate surface, the method comprising:

   a) providing a substrate with a surface to be coated;
   b) providing a biomimetic surfactant;
   c) mixing the biomimetic surfactant in a solvent mixture comprised of water and an organic water miscible solvent, thereby creating biomimetic surfactant solution;
   d) applying a first layer to the substrate surface;
   e) applying the biomimetic surfactant solution to the first layer, wherein the organic water miscible solvent drives the biomimetic surfactant out of solution onto the substrate surface creating a coating of biomimetic surfactant; and
   f) applying a heat treatment to the biomimetic coating.

10. The method of claim 9, wherein the biomimetic material is a surfactant selected from the group consisting of poly (N-vinyldextran aldonamide-co-N-vinylhexanamide)(PNVDA-co-PNVH), poly(N-vinyl dextran aldonamide-co-N-vinyl dodecano-amide) (PNVDA-co-PNVL), poly (N-vinyl hexyl amine-co-N-vinyl heparinamine) (PNVHA-co-PNVHep A), poly(N-vinyl hexyl amine-coN-vinyl heparinamine-co-N-vinyl maltonoamide)(PNVHA-co-PN-VHepA-co-PNVM), poly (N-vinyl-5-peptidyl-pentylamine-co-N-vinyldextran aldon- amine-co-N-vinyl hexyl amine (PVAm(Pep:Dex: Hex)), and poly ($N$-vinyldextranaldonamide-$co$-$N$-vinylperfluoroundec anamide).

11. The method of claim 9, wherein the substrate surface is selected from the group consisting of stainless steel, MP35N, titanium, gold, platinum, palladium, silver, and combinations thereof.

12. The method of claim 9, wherein the first layer is selected from the group consisting of polyurethane, silicon, polyether block amide (PEBA), polytetrafluoroethylene, polycarbonate, and polyetheretherketone.

13. The method of claim 9, wherein the solvent mixture comprises 40 to 60 volume percent organic water miscible solvent; preferably wherein the organic water miscible solvent is selected from the group consisting of isopropyl alcohol, methanol, ethanol, 1-propanol, 2-propanol, acetonitrile, $N,N'$-dimethylformamide and tetrahydrofuran.

14. The method of claim 9, wherein the concentration of the biomimetic surfactant is from 0.1 to 20 weight percent.

15. The method of claim 9, wherein the biomimetic coating is heat treated at 30°C to 55°C for 30 to 60 minutes.

FIG. 1

PNVDA-co-PNVH

PNVDA

PNVH

| STEP 1 | Clean substrate surface. |
|---|---|
| STEP 2 | Prepare biomimetic coating solution by dissolving the biomimetic surfactant in a water miscible solvent/water mixture. |
| STEP 3 | Apply biomimetic solution to substrate surface and dry. |
| STEP 4 | Clean coated substrate surface. |
| STEP 5 | Heat treat biomimetic coated surface. |

FIG. 2

Europäisches
Patentamt

European
Patent Office

Office européen
des brevets

## EUROPEAN SEARCH REPORT

Application Number

EP 10 16 5564

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/55742 A1 (MARCHANT ROGER E [US]; ZHANG TIANHONG [US]; QIU YONGXING [US]; RUEGSEG) 4 November 1999 (1999-11-04) * page 15, lines 23-34; figures 1,2,3; examples 8-10 * * page 5, lines 12-30 * | 1-15 | INV. A61L27/34 A61L29/08 A61L31/10 A61L33/00 A61L33/08 |
| X,D | US 2008/262614 A1 (MARCHANT ROGER E [US] ET AL) 23 October 2008 (2008-10-23) * paragraphs [0034] - [0038]; figures 1,2,3; examples 8-10 * | 1-15 | |
| X | US 2002/115836 A1 (TSANG RAY [US] ET AL) 22 August 2002 (2002-08-22) * paragraphs [0034], [0038], [0044] - [0051], [0078], [0079], [0089] - [0093], [0097]; examples 1-4; table 1 * | 1,3-8 | |
| A | J. M. H. M. SCHEUTJENS, G. J. FLEER: "Statistical theory of the adsorption of interacting chain molecules. 1. Partition function, segment density distribution, and adsorption isotherms" J. PHYS. CHEM., vol. 83, no. 12, 30 June 1979 (1979-06-30) , pages 1619-1635, XP002595452 DOI: 10.1021/j100475a012 * page 1629, left-hand column, last paragraph - page 1630, last paragraph; figures 5-7 * | 1-15 | TECHNICAL FIELDS SEARCHED (IPC) A61L C08B |

-/--

The present search report has been drawn up for all claims

| Place of search | Date of completion of the search | Examiner |
|---|---|---|
| Munich | 13 August 2010 | Schmitt, Johannes |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 16 5564

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EVGENI POPTOSHEV,BJOERN SCHOELER,FRANK CARUSO: "Influence of Solvent Quality on the Growth of Polyelectrolyte Multilayers" LANGMUIR, 7 January 2004 (2004-01-07), pages 829-834, XP002595453 online DOI: 10.1021/la035485u * page 829, right-hand column, last paragraph - page 832, left-hand column, last paragraph; figures 1,2 * | 1-15 | |

TECHNICAL FIELDS
SEARCHED (IPC)

The present search report has been drawn up for all claims

| Place of search | Date of completion of the search | Examiner |
|---|---|---|
| Munich | 13 August 2010 | Schmitt, Johannes |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

## ANNEX TO THE EUROPEAN SEARCH REPORT
## ON EUROPEAN PATENT APPLICATION NO.

EP 10 16 5564

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2010

| Patent document cited in search report | | Publication date | Patent family member(s) | | Publication date |
|---|---|---|---|---|---|
| WO 9955742 | A1 | 04-11-1999 | AT 437900 T | | 15-08-2009 |
| | | | EP 1086149 A1 | | 28-03-2001 |
| | | | ES 2330694 T3 | | 14-12-2009 |
| US 2008262614 | A1 | 23-10-2008 | NONE | | |
| US 2002115836 | A1 | 22-08-2002 | US 2002160098 A1 | | 31-10-2002 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61186524 A
- US 6759388 B, Marchant
- US 7276474 B
- US 20080247988 A
- US 20080262614 A
- US 776095 A